# EUROPEAN PATENT APPLICATION

(11) **EP 2 826 492 A1**
(43) Date of publication of application: **21.01.2015**
(21) Application number: 13761996.1
(22) Date of filing: 12.03.2013
(51) Int. Cl.: A61K 47/40, A61K 47/48, A61K 47/30, A61K 31/337

(54) **DRUG DELIVERY CONJUGATE CAPABLE OF CONTROLLED RELEASE, AND USE THEREOF**

(30) Priority: 13.03.2012 KR 20120025806
(71) Applicant: Postech Academy-Industry Foundation, Pohang-si, Gyeongsangbuk-do 790-784 (KR); Kyungpook National University Industry-Academic Cooperation Foundation, Daegu 702-701 (KR)
(72) Inventor: KIM, Won Jong, Pohang-si Gyeongsangbuk-do 790-751 (KR); NAMGUNG, Ran, Yongin-si Gyeonggi-do 448-160 (KR); LEE, Byung Heon, Daegu 706-020 (KR); KIM, In San, Daegu 706-939 (KR); HOFFMAN, Allan, Daegu 700-422 (KR)
(74) Representative: Towler, Philip Dean
(86) International application number: PCT/KR2013/001988
(87) International publication number: WO 2013/137627

(57) **Abstract**

The present invention relates to a hydrophobic drug delivery conjugate, to which cyclodextrin, poly(maleic anhydride), and a hydrophobic drug are bonded, and to a pharmaceutical composition comprising the hydrophobic drug delivery conjugate as an active ingredient. The hydrophobic drug delivery conjugate according to the present invention can effectively control the release rate and the delivery rate of a hydrophobic drug by regulating the physicochemical bonding and/or composition of the cyclodextrin, poly(maleic anhydride), and hydrophobic drug, and can also increase the effect of the drug by significantly increasing the solubility of the hydrophobic drug. Further, the present invention can enable the type of the hydrophobic drug to be varied, as well as a peptide having target directivity to be introduced into a surface of the conjugate, such that the present invention can be applied to the treatment of various diseases in addition to cancer treatment.

## Description

### [Technical Field]

The present invention relates to a drug delivery conjugate capable of controlled release, and a pharmaceutical composition including the same.

### [Background Art]

A tumor is a product of abnormal, uncontrolled, and disorganized cell growth caused by an excess of abnormal cells. Such a tumor is classified into a malignant tumor when it shows destructive proliferation, infiltration, and metastasis. In particular, the tumor may be referred to as a genetic disorder developed by mutation of a gene in an aspect of molecular biology. It is known that each tumor averagely has 50 to 80 mutant genes not present in normal cells. Therefore, the cancer incidence rate may increase in an aging society. Accumulation of mutated genes results in an increase in probability of mutating aged cells into cancer cells. Also, in Korea, 100,000 cancer patients were newly developed in 1999, and increased to over 180,000 in 2008 (reported by the Ministry of Health and Welfare in 2010). Accordingly, ardent research has been conducted to develop an effective anticancer drug.

A representative example of the anticancer drug is a paclitaxel preparation. Paclitaxel is a representative natural drug that is isolated from the husk of Pacific yew and widely used. When introduced into the cells, paclitaxel is known to induce growth of normal microtubules in a cell division process so that the cells can be arrested in the G2-M phase, inhibit the cell division, and finally induce apoptosis. Also, paclitaxel responds to various cancers, and thus has been used as a therapeutic agent for treating various cancers such as breast carcinoma, lung cancer, ovarian cancer, cervical carcinoma, and the like. However, since paclitaxel is a hydrophobic drug that is restrictively applicable due to low solubility, that is, poor solubility, in an aqueous solution, organic solvents such as Cremophor EL and ethanol have been used to solubilize paclitaxel. In this case, side effects may be caused by use of the organic solvent.

In this regard, Cremophor EL was used as a conventional solvent to increase the solubility of paclitaxel. Cremophor EL is polyoxyethylated castor oil that aids in dissolving paclitaxel in water, but has side effects to cause severe anaphylactoid reactions, hypersensitivity, hyperlipidaemia, abnormal lipoprotein patterns, erythrocyte aggregation, irreversible peripheral neuropathy, and the like (Gelderblom, H., Verweij, J., Nooter, K., and Sparreboom, A. (2001) Cremophor EL: the drawbacks and advantages of vehicle selection for drug formulation. Eur. J. Cancer 37, 1590-1598., Weiss, R. B., Donehower, R. C., Wiernik, P. H., Ohnuma, T. Gralla, R. J., Trump, D. L.; Baker Jr, J. R., Van Echo, D. A., Von Hoff, D. D., and Leyland-Jones, B. (1990) Hypersensitivity reactions from taxol. J. Clin. Oncol., 8, 1263-1268.; Lorenz, W., Riemann, H. J., and Schmal, A. (1997) Histamine release in dogs by Cremophor EL and its derivatives: oxyethylate oleic acid is the most effective constituent. Agents Actions 7, 63-67). Therefore, there is a need for development of new methods of increasing solubility of a paclitaxel preparation.

To effectively treat cancer using the hydrophobic drug such as paclitaxel as described above, development of drug delivery systems which have no side effects by a solvent, show target directivity to cancer cells, and can increase solubility of a hydrophobic drug is often required.

### [Disclosure]

### [Technical Problem]

Therefore, the present invention is designed to solve the problems of the prior art, and therefore it is an object of the present invention to provide a drug delivery conjugate to which a biocompatible polymer (i.e., cyclodextrin), poly(maleic anhydride), and a hydrophobic drug are bonded to improve solubility of the hydrophobic drug, and a pharmaceutical composition including the drug delivery conjugate as an active ingredient.

However, the technical objects of the present invention are not limited thereto, and other objects of the present invention which are not disclosed herein will become more apparent to those of ordinary skill in the art by describing in detail exemplary embodiments thereof.

### [Technical Solution]

According to an aspect of the present invention, there is provided a hydrophobic drug delivery conjugate to which cyclodextrin, poly(maleic anhydride), and a hydrophobic drug are bonded.

According to one exemplary embodiment of the present invention, in the hydrophobic drug delivery conjugate, the cyclodextrin and the poly(maleic anhydride) may be bonded to each other via ester bonds.

According to another exemplary embodiment of the present invention, in the hydrophobic drug delivery conjugate, the hydrophobic drug and the poly(maleic anhydride) may be bonded to each other via ester bonds.

According to still another exemplary embodiment of the present invention, the hydrophobic drug delivery conjugate may be prepared so that the hydrophobic drug can be included in the cyclodextrin.

According to still another exemplary embodiment of the present invention, the hydrophobic drug delivery conjugate may be prepared in the form of nano-sized particles.

According to still another exemplary embodiment of the present invention, the hydrophobic drug delivery conjugate may bond a peptide having target directivity to cancer cells.

According to still another exemplary embodiment of the present invention, the peptide may be a peptide having cysteine introduced into the N-terminus thereof.

According to yet another exemplary embodiment of the present invention, the hydrophobic drug may be paclitaxel.

According to another aspect of the present invention, there is provided a pharmaceutical composition for treatment of cancer including the hydrophobic drug delivery conjugate as an active ingredient.

According to one exemplary embodiment of the present invention, the cancer may be selected from the group consisting of breast carcinoma, lung cancer, ovarian cancer, cervical carcinoma, and colorectal adenocarcinoma.

### [Advantageous Effects]

The hydrophobic drug delivery conjugate according to one exemplary embodiment of the present invention can be useful in effectively controlling the release and delivery rates of a hydrophobic drug by regulating physical/chemical bonds and/or compositions of the cyclodextrin, the poly(maleic anhydride), and the hydrophobic drug, and can also be useful in improving the effects of the hydrophobic drug by significantly increasing the solubility of the hydrophobic drug. Also, the hydrophobic drug delivery conjugate is expected as original technology applicable to treatment of various diseases as well as treatment of cancer since the types of hydrophobic drug can be widely varied and a peptide having target directivity can be easily introduced into a surface of the conjugate.

### [Description of Drawings]

FIG. 1 is a diagram schematically showing the concept of a drug delivery conjugate.
FIG. 2 is a diagram schematically showing a method of preparing a drug delivery conjugate.
FIG. 3 is a schematic diagram showing a structure of the drug delivery conjugate.
FIG. 4 is a diagram showing the ¹H-NMR spectrum results of the drug delivery polymer, as observed by ¹H-NMR spectroscopy.
FIG. 5 is a diagram showing the ¹H-NMR spectrum results of the drug delivery polymer, as observed by ¹H-NMR spectroscopy.
FIG. 6 is a diagram showing the drug delivery conjugate, as determined using dynamic light scattering (DLS).
FIG. 7 is a diagram showing the collapse of the drug delivery conjugate by free cyclodextrin.
FIG. 8 is a diagram showing the results obtained by measuring the solubility of paclitaxel.
FIG. 9 is a diagram showing the results obtained by determining an ability of the drug delivery conjugate to release paclitaxel.
FIG. 10 is a diagram showing the results obtained by determining an anticancer effect of the drug delivery conjugate in cervical carcinoma cells.
FIG. 11 is a diagram showing the results obtained by determining an anticancer effect of the drug delivery conjugate in lung cancer cells.
FIG. 12 is a diagram showing the results obtained by determining an anticancer effect of the drug delivery conjugate in breast carcinoma cells.
FIG. 13 is a diagram showing the results obtained by determining an anticancer effect of the drug delivery conjugate in colorectal adenocarcinoma cells.
FIG. 14 is a diagram schematically showing a method of bonding a peptide having target directivity to cancer to the drug delivery conjugate.
FIG. 15 is a diagram schematically showing the synthesis of a poly-x-CD::poly-x-PTX/FCR/AP-1 conjugate and a diagram showing the ¹H-NMR spectrum results of the conjugate.
FIG. 16 is a diagram showing the results obtained by monitoring the kinetics of AP-1 by observing the absorbance of released 2-pydidinethione at 370 nm while conjugating the AP-1 peptide.

### [Best Mode]

The present inventors have conducted ardent research on a drug delivery system which has target directivity to cancer cells and can increase solubility of the hydrophobic drug. Therefore, the present invention has been completed based on these facts.

The present invention provides a drug delivery conjugate to which cyclodextrin (CD), poly(maleic anhydride), and a hydrophobic drug are bonded.

The present inventors have made an attempt to develop a drug delivery system causing no toxicity to cells, and thus used a biocompatible polymer (i.e., cyclodextrin) and poly(maleic anhydride). The poly(maleic anhydride) (PolyMALEIC) is a copolymer obtained by polymerizing a maleic anhydride with another monomer. In this case, since an anhydride group of the poly(maleic anhydride) is highly reactive to an amine group or a hydroxyl group, monomers containing an amine group or a hydroxyl group as a functional group may be easily introduced into the main chain of the polymer. Also, as a ring of the anhydride group is converted into a carboxyl acid group during such a conjugation process, solubility of the polymer in an aqueous solution may be enhanced. In the present invention, a polymer is prepared by bonding the hydrophobic drug or the cyclodextrin to the poly(maleic anhydride) via a cleavable ester bond, based on such characteristics. Types of the hydrophobic drug capable of being bonded to the poly(maleic anhydride) are not particularly limited. Preferably, the hydrophobic drug is paclitaxel.

Meanwhile, cyclodextrins are molecules that consist of 6 to 8 monosaccharides which are linked in a cyclic manner, and have a conical structure, and are classified into α-, β- and γ-cyclodextrins. Cyclodextrins may be used stably in living organisms without any toxicity since they are composed of monosaccharides. Also, cyclodextrins are known to have hydrophilic properties when OH groups are exposed to external environments, and have hydrophobic properties in internal spaces thereof. As a result, the cyclodextrins may support hydrophobic materials due to the presence of the internal spaces having such hydrophobic properties, and also enhance solubility of the hydrophobic drug due to an interaction with the hydrophobic drug. In the present invention, a drug delivery conjugate to which cyclodextrin, poly(maleic anhydride), and a hydrophobic drug are bonded is prepared by including the hydrophobic drug in the cyclodextrin to form an inclusion complex, based on such characteristics. Such a conjugate may locally restrict movement of the two polymers, and thus may be in the form of a nanogel similar to a gel or having a cross-linked nature. For the conjugate, types of the hydrophobic drug capable of being included in the cyclodextrin are not particularly limited. Preferably, the hydrophobic drug is paclitaxel, or the like.

Only the hydrophobic drug may be dissociated from the drug delivery conjugate in the form of a nanogel prepared by the inclusion in a state in which the conjugate is not cleaved, and a cyclodextrin-hydrophobic drug polymer may be released by cleavage of the ester bond. In this case, the drug release may be controlled by adjusting an association-dissociation constant of the inclusion complex as one of the main kinetic parameters for controlling the drug release behavior. The release rate of the drug may decrease when the hydrophobic drug is strongly bonded to the cyclodextrin, whereas the release rate of the drug may increase when the hydrophobic drug is weakly bonded to the cyclodextrin. Therefore, the drug delivery conjugate according to one exemplary embodiment of the present invention may adjust the release and delivery rates of the drug by adjusting a hydrolysis rate of the ester bond between the poly(maleic anhydride) and the cyclodextrin and/or hydrophobic drug and a dissociation rate of the inclusion complex through a change in compositions of a formulation.

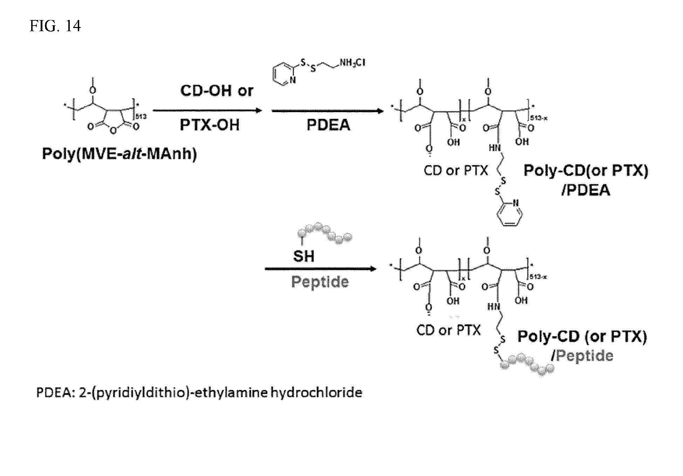

According to one exemplary embodiment of the present invention, it was confirmed that a peptide was able to be bonded to the drug delivery conjugate according to one exemplary embodiment of the present invention (Examples 2 to 6). Therefore, the peptide having cysteine introduced into the N-terminus thereof to show target directivity to cancer cells may be prepared and bonded to the drug delivery conjugate according to one exemplary embodiment of the present invention. A schematic diagram of a bonding method is shown in FIG. 14. Types of the peptide are not particularly limited. Preferably, the peptide may include an AP-1 peptide binding to interleukin-4 (IL-4) to specifically bind to breast carcinoma cells, a DUP-1 peptide specifically binding to prostate-specific membrane antigen (PSMA)-free cells (PC-3) in prostate cancer cells, an RGD peptide binding to integrin overexpressed in cancer cells, an NGR peptide binding to a CD 13 receptor present in new blood vessels around the cancer cells, and the like.

According to another exemplary embodiment of the present invention, it was also confirmed that the drug delivery conjugate effectively increased the solubility of the hydrophobic drug, paclitaxel, 4 times or more, and that the drug delivery conjugate decreased the inhibitory concentration of 50% (IC₅₀) of paclitaxel at least 4 times up to 150 times according to the type of cancer (see Example 2).

From these results, the drug delivery conjugate according to one exemplary embodiment of the present invention may increase solubility of the hydrophobic drug included in the cyclodextrin, and thus is expected to be applicable to treatment of various diseases. Therefore, the present invention provides a pharmaceutical composition including an effective amount of the drug delivery conjugate.

The pharmaceutical composition according to one exemplary embodiment of the present invention may include a pharmaceutically available carrier. The pharmaceutically available carrier may include a physiological saline solution, polyethylene glycol, ethanol, a vegetable oil, and isopropyl myristate, but the present invention is not limited thereto.

Another aspect of the present invention provides a method of treating a disease, which includes administering a therapeutically effective amount of the pharmaceutical composition including the drug delivery conjugate as an active ingredient to a subject. In the present invention, the term "subject" refers to a target in need of treatment of a disease, and, more particularly, to a mammal such as a human or non-human primate, a mouse, a rat, a dog, a cat, a horse, or cattle. Also, in the present invention, the term "therapeutically effective amount" may be adjusted to a wide extent according to the body weight, age, gender, and health condition of a patient, a diet, an administration time, a method of administration, an excretion rate, and the severity of a disease, as apparent to those skilled in the related art.

The preferred dose of the pharmaceutical composition according to one exemplary embodiment of the present invention may vary according to the health condition and body weight of a patient, the severity of a disease, the type of a drug, a route of administration, and an administration time, but may be properly chosen by those skilled in the related art. However, preferably, the pharmaceutical composition may be administered daily at a dose of 0.001 to 100 mg/kg, and more preferably a dose of 0.01 to 30 mg/kg. The pharmaceutical composition may be administered once a day, or administered in divided doses. The drug delivery conjugate according to one exemplary embodiment of the present invention may be present at a dose of 0.0001 to 10% by weight, preferably 0.001 to 1% by weight, based on the total weight of the composition.

The pharmaceutical composition according to one exemplary embodiment of the present invention may be administered into mammals such as mice, rats, domestic animals, and humans through various routes of administration. A method of administration is not particularly limited. For example, the pharmaceutical composition may be administered orally, or rectally, or by intravenous, intramuscular, subcutaneous, cervical epidural, or intra-cerebroventricular injection.

### [Mode for Invention]

Hereinafter, the present invention will be described with reference to the following Examples in order to facilitate a better understanding of the present invention. However, it should be understood that the following Examples are given by way of illustration of the present invention only, and are not intended to limit the scope of the present invention.

### [EXAMPLES]

### Example 1: Preparation of drug delivery conjugate

To prepare a polymer to which cyclodextrin and poly(maleic anhydride) were bonded, OH groups of β-cyclodextrin (1 g) consisting of 7 monosaccharides were activated with lithium hydride (LiH; 7.0 mg) for 12 hours (under an anhydrous N₂ condition and in the presence of dimethylformamide (DMF; 20 mL)), and the resulting reaction solution was then added to a poly(maleic anhydride) (Poly(IB-*alt*-MAnh)) solution (90 mg/10 mL of DMF), reacted for 12 hours, purified through dialysis (MWCO 3,500), and freeze-dried to obtain a poly-x-CD polymer (poly(IB-*alt*-Manh)-g-CD_{b}) to which cyclodextrin and poly(maleic anhydride) were bonded. The conjugation ratio of the cyclodextrin and the poly(maleic anhydride) may be widely adjusted by adjusting an amount of the cyclodextrin (1 g or 0.5 g).

To prepare a polymer to which paclitaxel and poly(maleic anhydride) were bonded, OH groups of paclitaxel (33.3 mg) were activated with LiH (1 mg) for 12 hours (under an anhydrous N₂ condition and in the presence of dimethylformamide (DMF; 5 mL)), and the resulting reaction solution was added to a poly(maleic anhydride) (poly(MVE-*alt*-MAnh)) solution, reacted for 12 hours, purified through dialysis (MWCO 3,500), and freeze-dried to obtain a poly-x-PTX polymer (poly(MVE-*alt-*Manh)-*g*-PTX_{d}) to which paclitaxel and poly(maleic anhydride) were bonded. The conjugation ratio of the paclitaxel (PTX) and the poly(maleic anhydride) may be widely adjusted by adjusting an amount of the PTX (33.3 mg or 83.2 mg).

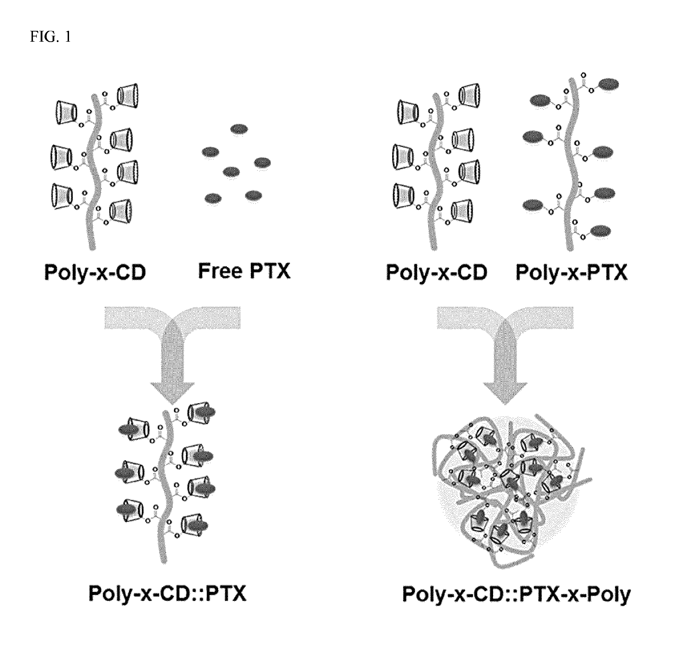

The scheme is shown in FIG. 1. The left panel of FIG. 1 is a schematic diagram showing the binding of free PTX to poly-x-CD, and the right panel of FIG. 1 is a schematic diagram showing the binding of poly-x-PTX to poly-x-CD.

The molecular weights and conjugation ratios of the poly-x-CD polymer and the poly-x-PTX polymer are shown in the following Table 1.

**[Table 1]**

| Denotation | Polymer | Grafted molecules | Feed ratio^{a} | Conj. ratio^{b} | Mw^{c} (g/mol) | Reacted MAnh^{d} (%) | w/w^{e} (%) |
|---|---|---|---|---|---|---|---|
| pCD₃ | Poly(IB-*alt*-Manh) | CD | 29 | 3 | 9 kDa | 7.7 | 36.2 |
| pCD₂₀ | Poly(IB-*alt*-Manh) | CD | 58 | 20 | 29 kDa | 51.3 | 79.1 |
| pPTX₇ | Poly(MVE-*alt-*Manh) | PTX | 20 | 7 | 86 kDa | 1.6 | 7.0 |
| pPTX₁₇ | Poly(MVE-*alt-*Manh) | PTX | 50 | 17 | 95 kDa | 3.3 | 15.4 |

| | | | | | | | |
|---|---|---|---|---|---|---|---|
| a. CD or PTX/polymer molar ratio b. CD or PTX/polymer molar ratio after purification; measured and calculated by ¹H-NMR c. Calculated molecular weights of polymer conjugates based on the conjugation ratio d. (Moles of reacted MAnh groups/moles of total MAnh groups per one polymer chain)×100 (%) e. (Weight of CD (or PTX)/weight of pCD (or pPTX))×100 (%) * Poly(IB-alt-MAnh) = approximately 6 kDa; poly(MVE-alt-MAnh) = approximately 80 kDa | | | | | | | |

To prepare a drug delivery conjugate, paclitaxel or a poly-x-PTX polymer was dissolved in ethanol, and a poly-x-CD polymer was dissolved in distilled water. Thereafter, the two resulting solutions were mixed at a CD:PTX molar ratio of 1:1, stirred for 12 hours so that CD and PTX were bonded to the drug delivery conjugate, and then freeze-dried to obtain a poly-x-CD::PTX conjugate (control), or a poly-x-CD::PTX-x-poly conjugate (a drug delivery conjugate). Then, the conjugates were dissolved in distilled water, and stored.

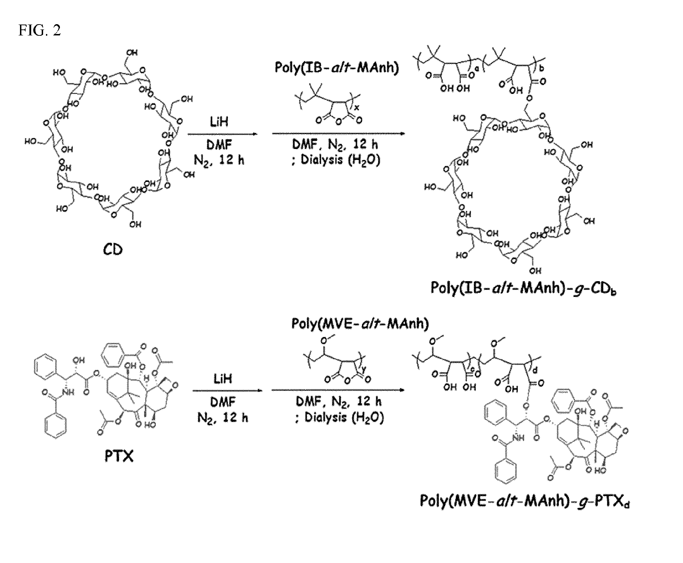

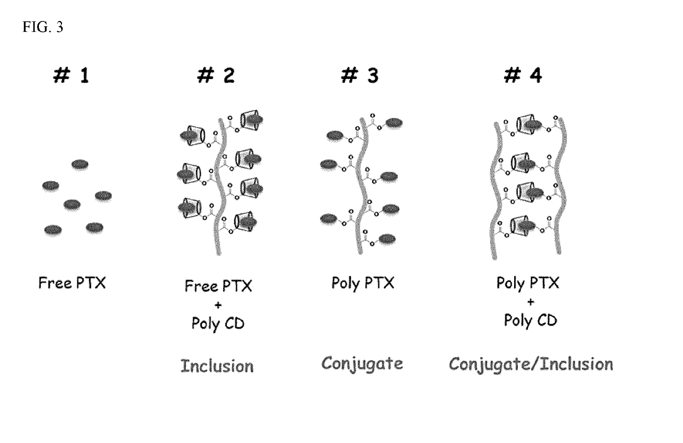

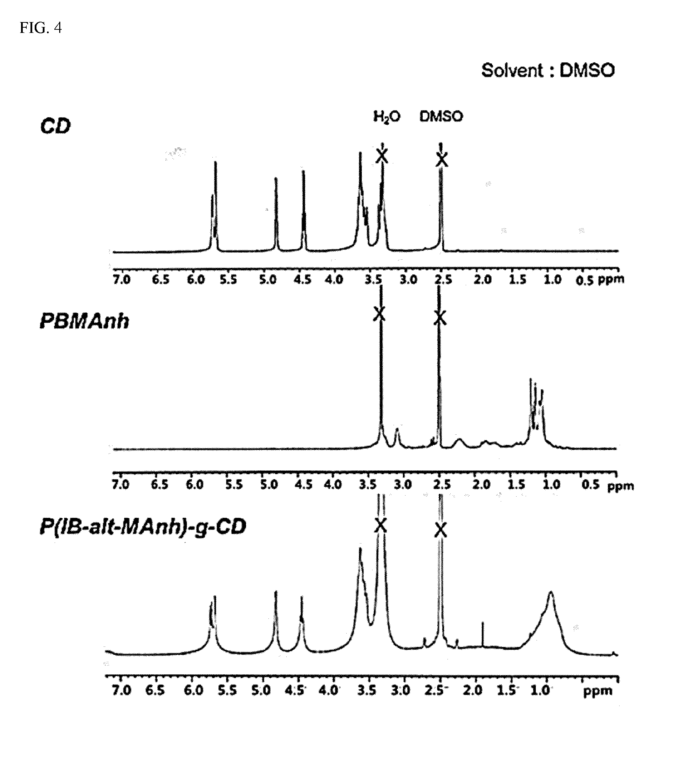

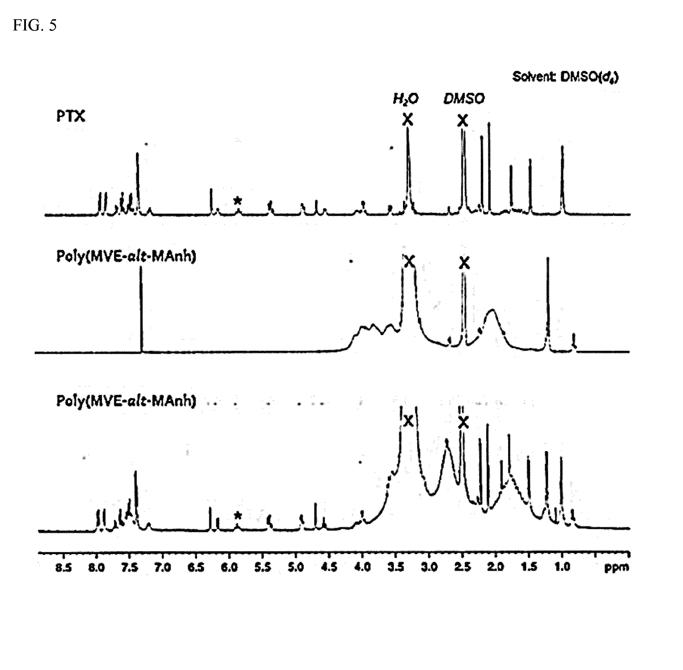

Schematic diagrams of this experiment are shown in FIGS. 2 and 3. The ¹H-NMR spectra of the prepared polymers and conjugates were observed using ¹H-NMR spectroscopy. The spectrum results are shown in FIGS. 4 and 5.

### Example 2: Characterization of drug delivery conjugate

### 2-1. Measurement of size of drug delivery conjugate

To measure the sizes and surface charges of the drug delivery conjugates prepared by the method of Example 1, the surface charges and sizes of the drug delivery conjugates in an aqueous solution were measured using a surface charge measurement system (Zetasizer Nano Z) and a size measurement system (Zetasizer Nano S). The measurement results are listed in the following Table 2.

**[Table 2]**

| No. | Samples | Size (d, nm) | Zeta potential (mV) |
|---|---|---|---|
| 1 | PTX | 806.4 ± 235.9 | -39.2 ± 1.50 |
| 2 | pCD::PTX | 420.3 ± 50.6 | -58.6 ± 2.62 |
| 3 | pPTX | 183.2 ± 12.6 | -41.7 ± 2.03 |
| 4 | pCD::pPTX | 54.6 ± 11.6 | -40.3 ± 0.77 |
| 5 | pCD | 243.7 ± 86.5 | -37.2 ± 1.78 |

As listed in Table 2, it was confirmed that the poly-x-CD::PTX-x-poly conjugate (#4) had an average diameter of approximately 55 nm, and thus had smaller diameter and size distribution than the paclitaxel (#1) and the poly-x-CD polymer (#5), both of which were present alone, indicating that the nanoparticles were effectively formed. Also, it was confirmed that polymer micelles having a diameter of approximately 183 nm were formed in the case of the poly-x-PTX polymer. On the other hand, it could be seen that the poly-x-CD::PTX conjugate had an average diameter of approximately 420 nm and a very wide size distribution, indicating that an aggregation occurred due to the low solubility.

Based on these results, it was confirmed that the poly-x-CD::poly-x-PTX (#4) drug delivery conjugate had the paclitaxel, the cyclodextrin, and the poly(maleic anhydride) bonded thereto, but had a diameter of approximately 50 nm and a narrow size distribution, indicating that the nanoparticles were stably formed and bonded to the drug delivery conjugate.

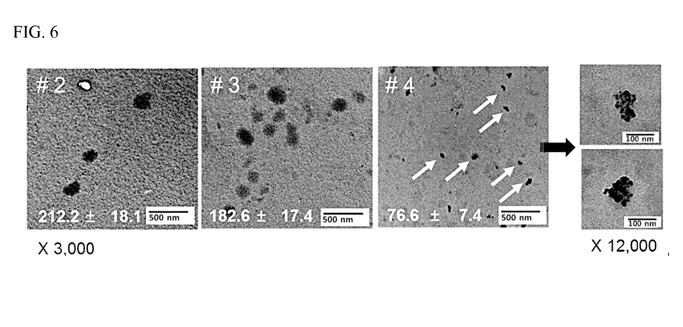

The same test sample was dried on a carbon grid, and the shape and size of the test sample were observed using a transmission electron microscope (TEM). The results are shown in FIG. 6.

As shown in FIG. 6, it could be seen that the poly-x-CD::PTX-x-poly conjugate (#4) had the smallest size, which was the same size of the particles as measured in an aqueous solution. Also, the spherical particles having a diameter of 200 nm or less were observed in the case of the test samples of the poly-x-CD::PTX conjugate (#2), the poly-x-PTX polymer (#3), and the poly-x-CD::PTX-x-poly conjugate (#4). Based on these results, it was confirmed that the drug delivery conjugate was a nanostructure having a diameter of approximately 200 nm, and was expected to be in the form of nanoparticles having a high density.

### 2-2. Determination of binding characteristics of drug delivery conjugate

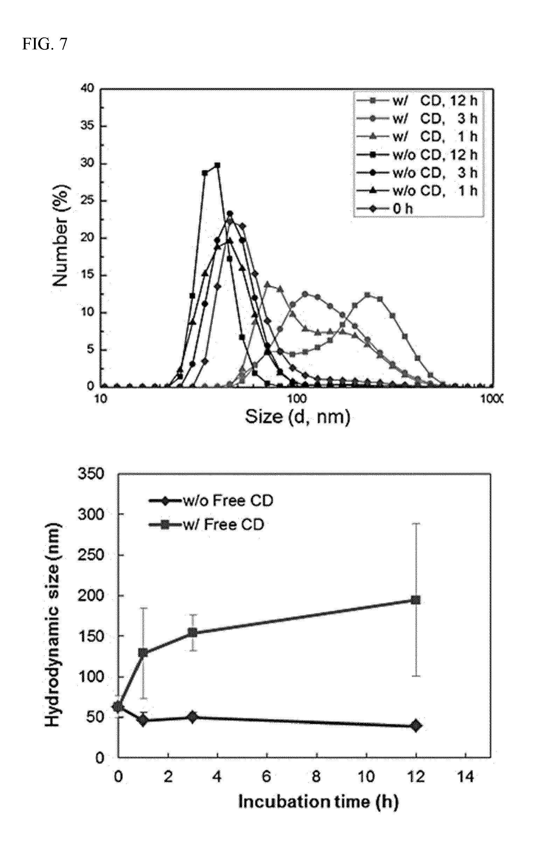

To determine whether the drug delivery conjugate (#4) prepared by the method of Example 1 formed an inclusion complex due to a characteristic structure formed between cyclodextrin and paclitaxel, cyclodextrin was added to a solution of the drug delivery conjugate (#4), and the size and number of the particles were measured at time points of 1 hour, 3 hours, and 12 hours using a size measurement system (Zetasizer Nano S). The measurement results are shown in FIG. 7.

As shown in FIG. 7, it was revealed that the size of the conjugate did not change with time when the cyclodextrin was not added, whereas the size of the conjugate gradually increased and the size distribution got wide when the cyclodextrin was added. These results showed that the shape of nanogels collapsed by an exchange reaction of the added cyclodextrin. Also, the results showed that the conjugate was a compound formed as paclitaxel was included in cyclodextrin.

### 2-3. Comparison of solubility of paclitaxel

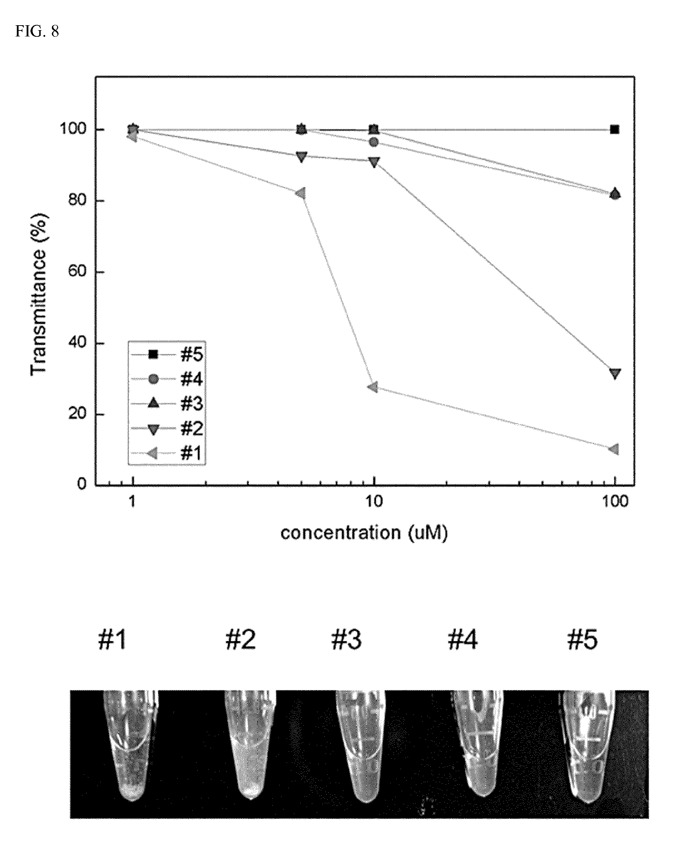

To compare the solubility of paclitaxel (PTX), the polymers and conjugates prepared by the method of Example 1 were measured for transmittance according to the concentrations of the polymers and conjugates using a ultraviolet (UV)-visible spectrometer (UV 2550, Shimadzu, Japan). The absorbance was measured at 500 nm at varying concentrations of PTX from 0 µM to 100 µM, and the transmittance was calculated using the absorbance. The results are shown in FIG. 8.

As shown in FIG. 8, it was revealed that the solubility of paclitaxel increased 4 times or more in the poly-x-PTX polymer (#3) and the poly-x-CD::PTX-x-poly conjugate (#4), compared to the paclitaxel (#1) which was present alone. Based on these results, it was confirmed that the drug delivery conjugate (#4) according to one exemplary embodiment of the present invention increased the solubility of paclitaxel.

### 2-4. Confirmation of release of paclitaxel

To confirm the release of paclitaxel, the polymers and conjugates prepared by the method of Example 1 were put into dialysis membranes (MWCO 3,500), and the dialysis membranes were put into vials containing a phosphate buffered saline (PBS) buffer solution (50 mL: pH 7.4 and pH 5.5), and incubated at 37°C for 50 hours. 25 mL of the buffer were drawn at constant time intervals, and 25 mL of a fresh solution was added. In this case, the buffer solution was extracted with 2 mL of DCM to collect the released paclitaxel. Then, the released paclitaxel was dissolved again in 500 µL of acetonitrile-water (50:50, v/v), and the concentration of PTX was analyzed using HPLC (a reverse-phase silica column (X-Terra MS C18, 4.6 mm×50 mm, 2.5 µm); a mobile phase of acetonitrile-water gradient pumped (LC-20AD, Shimadzu, Japan); a flow rate of 1.0 mL/min).

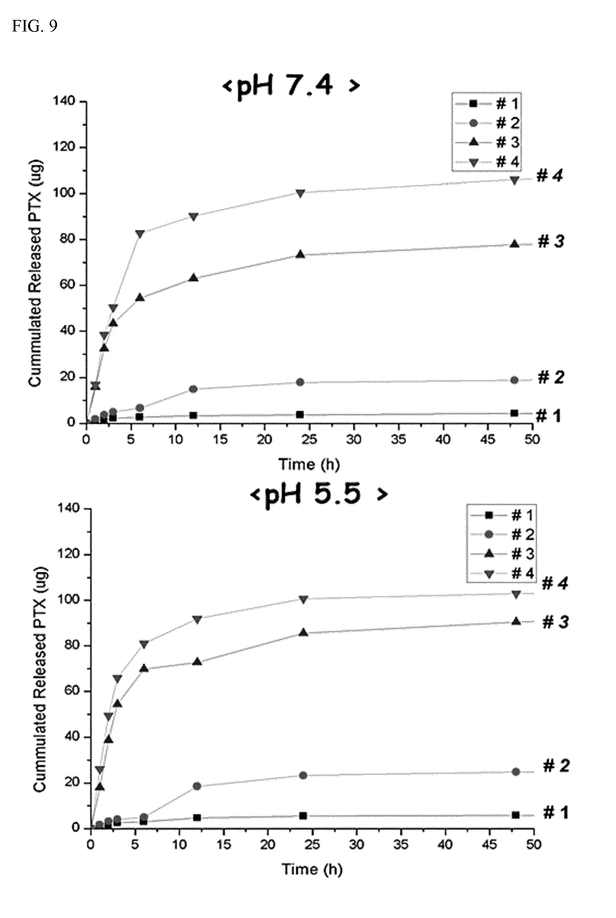

The test sample was injected at an amount of 200 µL, and the column effluent was detected at 227 nm (UV detector (SPD-20A, Shimadzu, Japan)). In this case, the calibration curve of paclitaxel appeared to be linear from 0.0025 mg/mL to 0.05 mg/mL. The results are shown in FIG. 9.

As shown in FIG. 9, it was revealed that the release rate and amount of paclitaxel were high at both pH values in the case of the poly-x-PTX polymer (#3) and the poly-x-CD::PTX-x-poly conjugate (#4). Based on these results, it was confirmed that the drug delivery conjugate according to one exemplary embodiment of the present invention was able to effectively release paclitaxel.

### 2-5. Measurement of anticancer effect

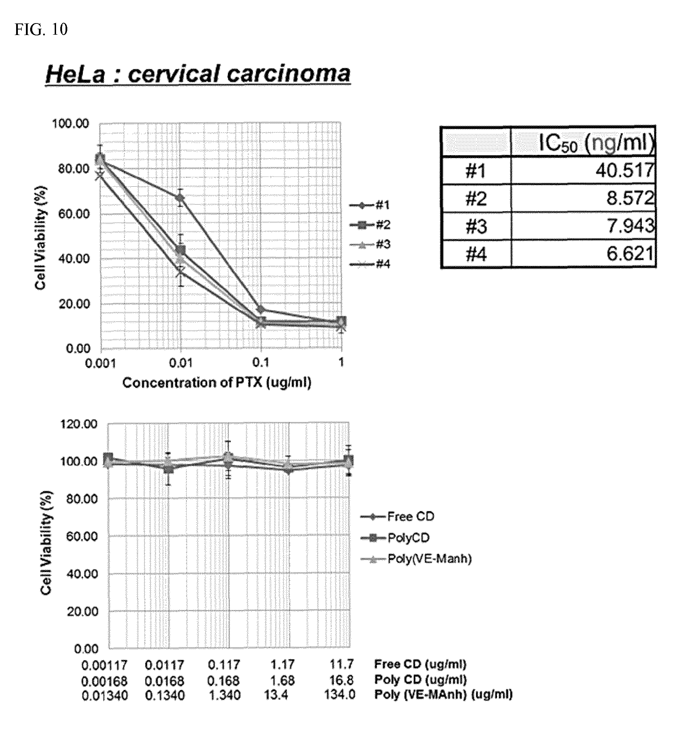

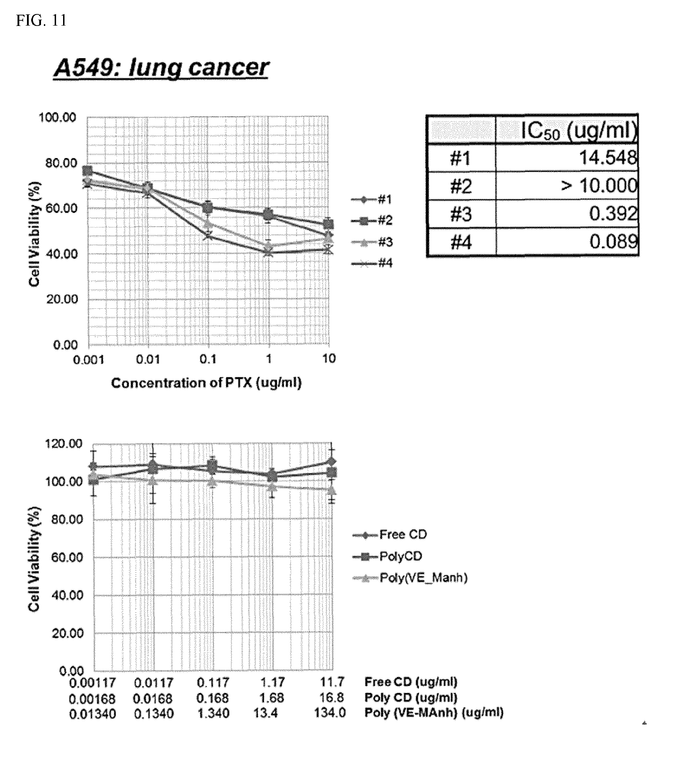

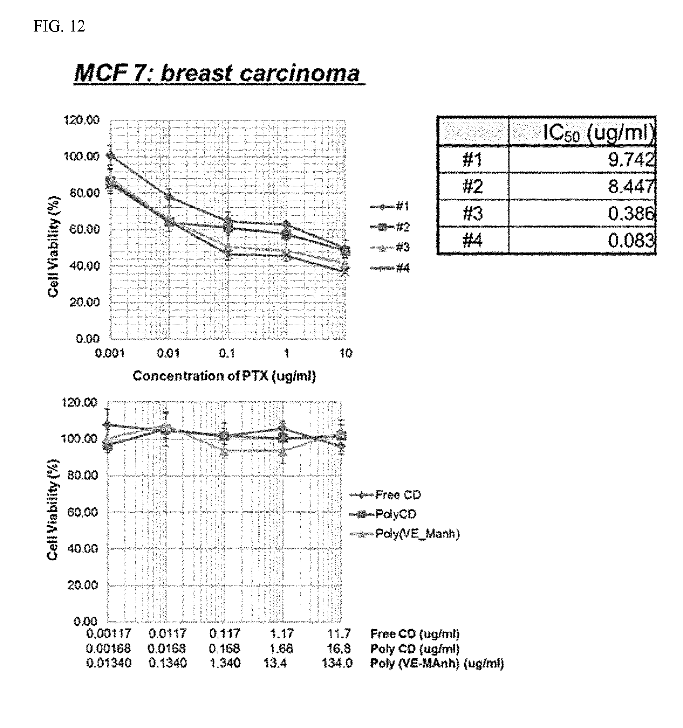

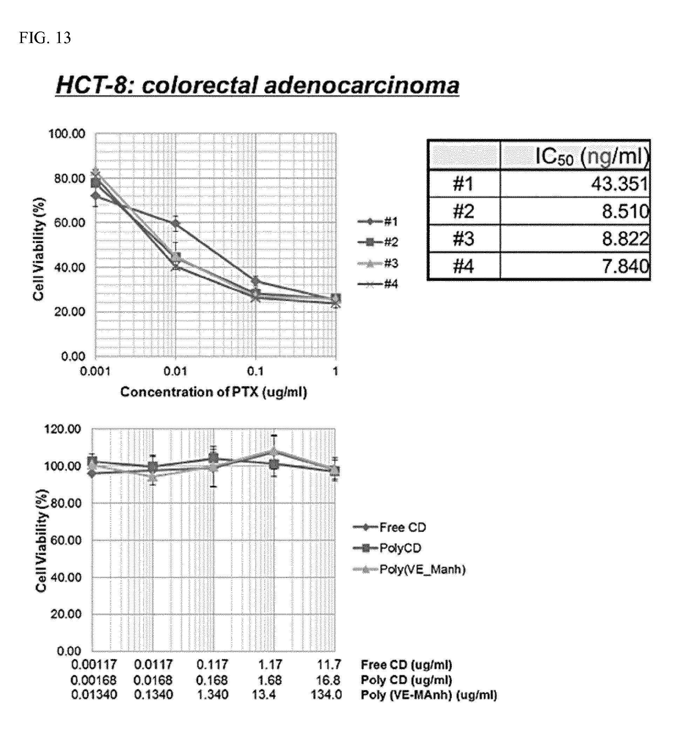

To measure an anticancer effect of the drug delivery conjugate, an MTT assay was used. Each of a HeLa cell line as a cervical carcinoma cell line, an A549 cell line as a lung cancer cell line, an MCF-7 cell line as a breast carcinoma cell line, and an HCT-8 cell line as a colorectal adenocarcinoma cell line were put into a 96-well plate at a dose of 5×10³ cells per well, and incubated at 37°C for 24 hours under a 5% CO₂ condition. Thereafter, the cells were treated with each of the polymers and conjugates prepared by the method of Example 1, and incubated at 37°C for 48 hours under a 5% CO₂ condition. Then, an MTT solution (20 µL, 5 mg/mL) was treated with a fresh medium, and the cells were incubated for 4 hours. Subsequently, the cells were treated with DMSO (150 µL), and measured for absorbance at 570 nm. The cell viability was calculated as a relative value on the assumption that the activity of the cells not treated with the test sample was set to 100%. The results are shown in FIGS. 10 to 13.

As shown in FIGS. 10 to 13, it was revealed that the drug delivery conjugate (#4) had the lowest IC₅₀ for all the cell lines, and that the concentration of the drug delivery conjugate was able to decrease at least 4 times up to 150 times.

Based on these results, it was confirmed that the drug delivery conjugate according to one exemplary embodiment of the present invention decreased the IC₅₀ in paclitaxel solution, and also increased the drug delivery efficiency, and thus was able to be used as an effective paclitaxel delivery system.

### 2-6. Synthesis of poly-x-CD::poly-x-PTX/FCR/AP-1 conjugate into which peptide was introduced (Scheme)

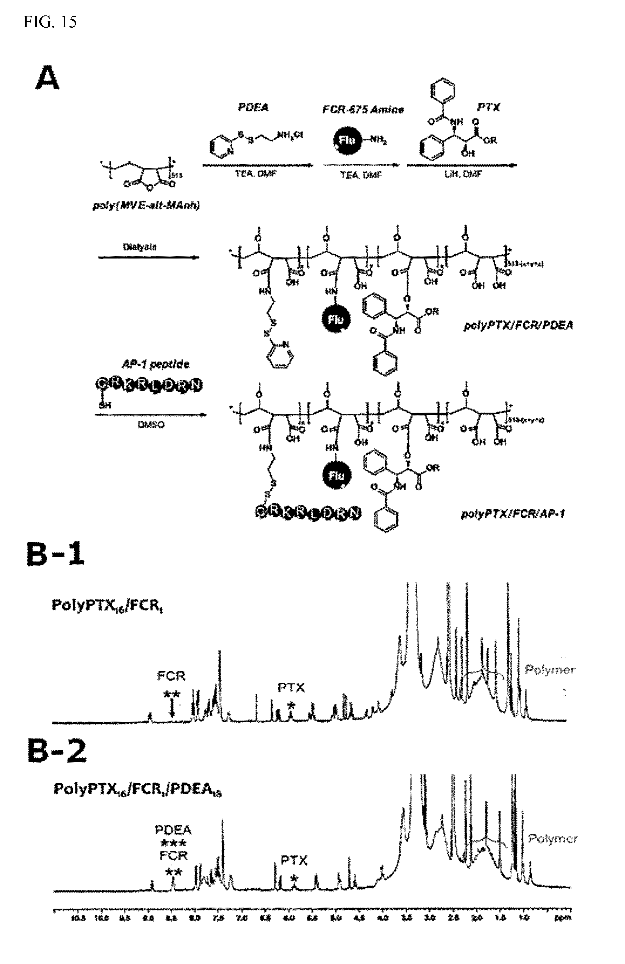

The poly-x-CD::poly-x-PTX/FCR/AP-1 conjugate was synthesized, as shown in FIG. 15A.

### (1) Synthesis of Poly-x-PTX/FCR/PDEA

9 mg of 2-(2-pydidinyldithio)ethane amine (PDEA) and 1.9 mg of FCR-675 amine were dissolved in 2 mL of DMF, and slowly added to 8 mL of a DMF solution in which 160 mg of Poly (MVE-alt-MAnh) treated with 10 µL of TEA was dissolved. The resulting mixture solution was then reacted for 12 hours. 33.6 mg of PTX activated with 5 mg of LiH was added to the solution, and further reacted for 12 hours. Thereafter, DMF was volatilized, and the mixture solution was dialyzed (MWCO 3,500) in water, purified, and then freeze-dried to obtain poly-x-PTX/FCR/PDEA. The ¹H-NMR spectroscopy results showed that 16 PTX molecules, one FCR molecule, and 16 PDEA molecules were conjugated to each polymer per one chain (poly-x-PTX₁₆/FCR₁/PDEA₁₈) (FIGS. 15B-1 and 15B-2).

### (2) Synthesis of Poly-x-PTX/FCR/AP-1

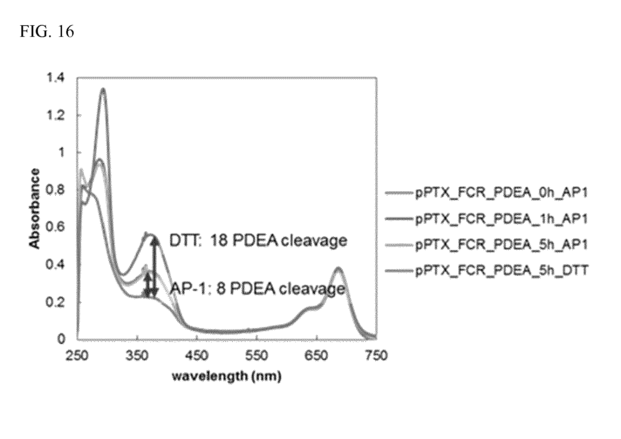

The experiment in which an AP-1 peptide having Cys introduced into the N-terminus thereof was conjugated to the poly-x-PTX₁₆/FCR₁/PDEA₁₈ was performed. 130 mg of the poly-x-PTX₁₆/FCR₁/PDEA₁₈ was dissolved in 15 mL of DMSO, and a solution (5 mL DMSO) containing 15 mg of AP-1 was slowly added to the resulting solution, and reacted for 24 hours. In this case, the absorbance of 2-pydidinethione released with progression of the reaction were measured at 370 nm, and the kinetics of AP-1 were monitored (FIG. 16). As a result, it was revealed that 8 AP-1 molecules were conjugated to the polymer so that the poly-x-PTX₁₆/FCR₁/AP-1₈ was synthesized. After the reaction, the poly-x-PTX₁₆/FCR₁/AP-1₈ was dialyzed (MWCO 3,500) in water, purified, and then freeze-dried to obtain a blue solid as an end product.

### (3) Synthesis of Poly-x-CD::Poly-x-PTX/FCR/AP-1

Solutions obtained by dissolving 22.0 mg of the Poly-x-CD20 (M_{w} 29 kDa) and 100 mg of the poly-x-PTX/FCR/AP-1 (Mw 105 kDa) in 5 mL of water, respectively, were mixed, incubated for 12 hours, and then freeze-dried to synthesize a Poly-x-CD::Poly-x-PTX/FCR/AP-1 conjugate.

Based on these results, it was confirmed that the drug delivery conjugate according to one exemplary embodiment of the present invention was able to be bonded to the peptide having cysteine introduced into the N-terminus thereof to show target directivity to cancer cells.

### [Industrial Applicability]

The hydrophobic drug delivery conjugate according to one exemplary embodiment of the present invention can be useful in effectively controlling the release and delivery rates of a hydrophobic drug by regulating physical/chemical bonds and/or compositions of the cyclodextrin, the poly(maleic anhydride), and the hydrophobic drug, and can also be useful in improving the effects of the hydrophobic drug by significantly increasing the solubility of the hydrophobic drug. Also, the hydrophobic drug delivery conjugate can be applied to treatment of various diseases as well as treatment of cancer since a peptide having target directivity can be easily introduced into a surface of the conjugate.

It will be apparent to those skilled in the art that various modifications can be made to the above-described exemplary embodiments of the present invention without departing from the spirit or scope of the invention. Thus, it is intended that the present invention covers all such modifications provided they come within the scope of the appended claims and their equivalents.

## Claims

1. A hydrophobic drug delivery conjugate to which cyclodextrin, poly(maleic anhydride), and a hydrophobic drug are bonded.

2. The hydrophobic drug delivery conjugate of claim 1, wherein in the hydrophobic drug delivery conjugate, the cyclodextrin and the poly(maleic anhydride) are bonded to each other via ester bonds.

3. The hydrophobic drug delivery conjugate of claim 1, wherein in the hydrophobic drug delivery conjugate, the hydrophobic drug and the poly(maleic anhydride) are bonded to each other via ester bonds.

4. The hydrophobic drug delivery conjugate of claim 1, wherein the hydrophobic drug delivery conjugate is prepared so that the hydrophobic drug is included in the cyclodextrin.

5. The hydrophobic drug delivery conjugate of claim 1, wherein the hydrophobic drug delivery conjugate is prepared in the form of nano-sized particles.

6. The hydrophobic drug delivery conjugate of claim 1, wherein the hydrophobic drug delivery conjugate bonds a peptide having target directivity to cancer cells.

7. The hydrophobic drug delivery conjugate of claim 6, wherein the peptide is a peptide having cysteine introduced into the N-terminus thereof.

8. The hydrophobic drug delivery conjugate of any one of claims 1 to 7, wherein the hydrophobic drug is paclitaxel.

9. A pharmaceutical composition for treatment of cancer comprising the hydrophobic drug delivery conjugate defined in any one of claims 1 to 7 as an active ingredient.

10. The pharmaceutical composition of claim 9, wherein the cancer is selected from the group consisting of breast carcinoma, lung cancer, ovarian cancer, cervical carcinoma, and colorectal adenocarcinoma.
